Europäisches Patentamt

European Patent Office

Office européen des brevets

(19)

(11) Veröffentlichungsnummer: **0 002 506**
**B1**

(12) **EUROPÄISCHE PATENTSCHRIFT**

(45) Veröffentlichungstag der Patentschrift: **16.09.81**

(21) Anmeldenummer: **78101615.9**

(22) Anmeldetag: **08.12.78**

(51) Int. Cl.³: **A 61 K 7/06,** A 61 K 7/08
//C08L5/08

(54) **Verwendung von Mitteln zur Kur sowie zum Waschen von Haaren.**

(30) Priorität: **09.12.77 DE 2754796**

(43) Veröffentlichungstag der Anmeldung:
**27.06.79 Patentblatt 79/13**

(45) Bekanntmachung des Hinweises auf die Patenterteilung:
**16.09.81 Patentblatt 81/37**

(84) Benannte Vertragsstaaten:
**DE FR GB IT NL**

(56) Entgegenhaltungen:
**FR - A - 2 137 684**
**GB - A - 458 815**
**GB - A - 458 839**
**US - A - 3 632 754**

(73) Patentinhaber: **Wella Aktiengesellschaft**
**Berliner Allee 65**
**D-6100 Darmstadt (DE)**

(72) Erfinder: **Konrad, Eugen**
**Mecklenburger Strasse 101**
**D-6100 Darmstadt (DE)**
Erfinder: **Gross, Paul**
**Forstweg 54**
**D-6100 Darmstadt (DE)**
Erfinder: **Mager, Herbert, Dr.**
**Beaumont 5**
**CH-1700 Fribourg (CH)**

Courier Press, Leamington Spa, England.

# 0 002 506

## Verwendung von Mitteln zur Kur sowie zum Waschen von Haaren

Die Erfindung bezieht sich auf die Verwendung von Mitteln zur Kur sowie zum Waschen von Haaren, wobei die Haare mit wäßrigen Zubereitungen mit einem Gehalt an wasserlöslichen Salzen von Chitosan behandelt werden.

Haarkurmittel dienen der Verbesserung der Haarkondition und sind üblicherweise Öl-in-Wasser-Emulsionen, die als Gerüstbestandteile ölige, halbfeste oder feste Substanzen, wie beispielsweise Paraffinöl, Vaseline, Wollfett, Wollfettalkohole, Fettsäureester und Kohlenwasserstoffwachse enthalten. Als Emulgatoren für solche Emulsionen werden normalerweise quaternäre Ammoniumverbindungen wie Oxyäthylalkylammoniumphosphate, Alkyltrimethylammoniumchloride, Dialkyldimethylammoniumchloride, Alkyldimethylbenzylammoniumchloride und Alkylpyridiniumchloride, allein oder in Kombination mit nichtionogenen Emulgatoren, verwendet. Auch wäßrige Lösungen und wäßrige Gele mit einem Gehalt an quaternären Ammoniumverbindungen finden häufig als Haarkurmittel Anwendung.

Haarwaschmittel enthalten üblicherweise anionische Tenside wie beispielsweise Fettalkoholsulfate, Fettalkoholäthersulfate, Fettsäuremonoglyceridsulfate, Alkylarylsulfate und Sulfosuccinate. Spezielle Haarwaschmittel sind solche Präparate, die als waschaktive Substanzen nichtionische, kationische, ampholytische Tenside oder Gemische aus diesen Tensiden enthalten. Zu den nichtionischen Tensiden sind beispielsweise Nonylphenolpolyglykoläther, Sorbitanpolyoxyalkylenfettsäureester, Fettsäureäthoxylate und Fettalkoholäthoxylate zu zählen. Vertreter der kationischen Tenside sind zum Beispiel Alkyldimethylbenzylammoniumchloride, Pentaoxyäthylammoniumchloride, Alkyltrimethylammoniumchloride, Alkyldimethylammoniumsaccharinate und Alkyldimethyldichlorbenzylammoniumchloride. Als ampholytische Tenside wären insbesondere Amidoalkylbetaine, Sulfobetaine und N-Alkyl-$\beta$-aminopropionsäuren anzuführen.

Es ist auch bekannt, zu Haarwaschmitteln geringe Mengen von Substanzen zur besseren Konditionierung des Haares zuzugeben. Neben verschiedenen unterschiedlichen Verbindungen werden für diesen Zweck häufig quaternäre Ammoniumverbindungen und oxäthylierte Alkylolamide empfohlen.

Wie sich in der Praxis jedoch gezeigt hat, können die Haarkur- und Haarwaschmittel nach dem Stand der Technik hinsichtlich ihrer haarkonditionierenden Wirkung, insbesondere was den Griff, Glanz sowie die Kämmbarkeit des Haares anbetrifft, noch nicht voll zufriedenstellen.

Der vorliegenden Erfindung liegt nun die Aufgabe zugrunde, die Verwendung von Mitteln vorzuschlagen, wodurch eine zufriedenstellende Kondition des Haares ermöglicht wird. Dies wird erfindungsgemäß dadurch erreicht, daß man Haarkur- und Haarwaschmittel mit einem Gehalt an wasserlöslichen Salzen von Chitosan verwendet. Das Ergebnis besteht in einer hervorragenden haarkonditionierenden Wirkung, wobei die Kämmbarkeit sowie der Griff und Glanz, insbesondere bei geschädigtem Haar, wesentlich verbessert werden.

Das Chitosan ist ein hochpolymeres Amin und befähigt, mit Säuren Salze zu bilden. Es wird durch alkalische Entacetylierung von Chitin hergestellt. Die vollständige Entacetylierung ist schwierig, weil das Alkali während der Reaktion nur unvollkommen in die Chitinpartikel eindringt. Ein praktisch acetylfreies Chitin, nämlich reines Chitosan, kann nur durch wiederholte Alkalibehandlung oder durch Fraktionierung erhalten werden. Das Chitosan des Handels stellt daher ein mehr oder weniger entacetyliertes Produkt mit einem Chitosan-Anteil von etwa 70 bis 90 Gew.% dar. Sowohl derartige Handelsprodukte als auch solche Produkte mit einem höheren Anteil von Chitosan sind erfindungsgemäß gleichermaßen mit Erfolg zu verwenden.

Durch Neutralisation der freien Aminogruppen des Chitosans mit Säuren lassen sich die entsprechenden Salze erhalten. Nach vorliegender Erfindung sind jedoch nur solche Salze verwendbar, die in Wasser löslich sind. Entsprechende Säuren sind beispielsweise Salzsäure, Ameisensäure, Essigsäure, Milchsäure, Glykolsäure, Malonsäure, Benzosäure, Adipinsäure, Nitrilotriessigsäure, Äthylendiamintetraessigsäure, p-Hydroxybenzoesäure, Citronensäure, Benzoldisulfonsäure, Salicylsäure und Chlorsulfonsäure. Für die erfindungsgemäßen Mittel haben sich die Salze der Ameisensäure, Essigsäure und Milchsäure als besonders geeignet erwiesen. Die Säuremenge soll zweckmäßigerweise so gewählt sein, daß sie zur Neutralisation der freien Aminogruppen des eingesetzten Chitosans ausreicht. So benötigt man beispielsweise, um 1 g Chitosan (mit 90% freien Aminogruppen) in wässrige Lösung zu bringen, 3,36 g 10%ige Essigsäure. Ein geringer Überschuß der bei der Neutralisation verwendeten Säure ist auf die Wirksamkeit der erfindungsgemäß verwendbaren Mittel ohne Einfluß.

Die erfindungsgemäß zu verwendenden Mittel stellen wäßrige Lösungen, Emulsionen oder Gele dar, die durch einen Gehalt an wasserlöslichen Salzen von Chitosan gekennzeichnet sind. Die Konzentration dieser Salze in den Mitteln soll vorzugsweise etwa 0,5 bis 6,0 Gew.% betragen, jedoch können je nach Zusammensetzung der Mittel mitunter auch höhere oder niedrigere Anteile vorteilhaft sein.

Die nach vorliegender Anmeldung zu verwendenden Haarkurmittel entsprechen ansonsten hinsichtlich ihrer übrigen Bestandteile den bekannten Mitteln dieser Art, wozu auf deren eingangs geschilderte Zusammensetzung verwiesen wird. Was die anmeldungsgemäß zu verwendenden Haarwaschmittel anbetrifft, so haben sich insbesondere solche mit einem Gehalt an nichtionischen, kationischen oder amphoteren Tensiden als geeignet erwiesen. Diese entsprechen den eingangs erwähnten speziellen Haarwaschmitteln.

Selbstverständlich können die anmeldungsgemäß verwendbaren Haarkur- und Haarwaschmittel neben den genannten Bestandteilen auch übliche kosmetische Zusätze wie beispielsweise Parfümöle, bakterizide und fungizide Stoffe, Trübungsstoffe, Verdicker und direkt auf das Haar aufziehende Farbstoffe enthalten.

Von den bekannten kosmetischen Farbstoffen, die einzeln oder in Mischung vorliegen können, seien beispielsweise die folgenden Klassen erwähnt: Aromatische Nitrofarbstoffe (z.B. 1,4-Diamino-2-nitrobenzol), Azofarbstoffe (z.B. Acid Brown 4), Anthrachinonfarbstoffe (z.B. C.I. Disperse Violet 4) und Triphenylmethanfarbstoffe (z.B. Basic Violet 1), wobei die Farbstoffe dieser Klassen je nach der Art ihrer Substituenten sauren, nichtionogenen oder basischen Charakter haben können. Ihre Gesamtkonzentration beträgt üblicherweise etwa 0,05 bis 2,0 Gew.%.

Die Anwendung der Mittel gemäß vorliegender Anmeldung erfolgt in der Weise, daß diese Mittel auf das Haar aufgebracht, etwa 3 bis 10 Minuten lang einwirken gelassen und anschließend wieder ausgespült werden. Zur Spülung wird vorzugsweise Wasser verwendet.

Die folgenden Beispiele sollen den Gegenstand der Erfindung näher erläutern, ohne ihn darauf zu beschränken.

<div align="center">Beispiele</div>

Haarkurmittel

<div align="center">Beispiel 1</div>

Emulsion, bestehend aus

| | | |
|---|---|---|
| 3,0 | g | Stearylalkohol |
| 1,0 | g | Adeps lanae |
| 1,0 | g | Vaselin |
| 1,0 | g | Chitosan (mit 90% freien Aminogruppen) |
| 0,76 | g | Ameisensäure, 10%ig |
| 2,0 | g | Tris-(oligooxyäthyl)-octadecyl-ammonium-phosphat, 50%ig |
| 91,94 | g | Wasser, vollentsalzt |
| 100,00 | g | |

<div align="center">Beispiel 2</div>

Emulsion, bestehend aus

| | | |
|---|---|---|
| 4,0 | g | Cetylstearylalkohol |
| 0,3 | g | Chitosan (mit 90% freien Aminogruppen) |
| 1,48 | g | Milchsäure, 10%ig |
| 2,5 | g | Kokospentaäthoxymethylammoniumchlorid |
| 1,0 | g | Sorbitanmonopalmitat mit 20 Mol Äthylenoxyd |
| 90,72 | g | Wasser, vollentsalzt |
| 100,00 | g | |

<div align="center">Beispiel 3</div>

Emulsion, bestehend aus

| | | |
|---|---|---|
| 5,0 | g | Cetylalkohol |

<div align="center">3</div>

3,0 g Lauryldimethyl-benzyl-ammoniumchlorid, 35%ig

1,2 g Dimethyl-carboxymethylen-propylenamidostearatbetain, 35%ig

0,45 g Chitosan (mit 90% freien Aminogruppen)

1,14 g Ameisensäure, 10%ig

89,21 g Wasser, vollentsalzt

100,00 g

Beispiel 4

Gel, bestehend aus

0,6 g Hydroxypropylmethylcellulose

2,1 g Chitosan (mit 90% freien Aminogruppen)

5,3 g Ameisensäure, 10%ig

0,5 g Laurylpyridiniumchlorid

91,5 g Wasser, vollentsalzt

100,0 g

Beispiel 5

Emulsion, bestehend aus

4,0 g Cetylalkohol

1,5 g Vaseline

2,5 g Tris-(oligooxyäthyl)-octadecyl-ammoniumphosphat, 50%ig

0,3 g Chitosan (mit 90% freien Aminogruppen)

0,75 g Ameisensäure, 10%ig

0,1 g C.I. Basic Violet 1

90,85 g Wasser, vollentsalzt

100,00 g

Jeweils 35 g der Haarkurmittel gemäß den Beispielen 1 bis 5 werden im gewaschenen Haar verteilt und nach einer Einwirkungszeit von 3 bis 5 Minuten mit Wasser wieder ausgespült. Als Ergebnis wird ein ausgezeichneter Griff, Glanz sowie Kämmbarkeit des Haares erhalten.

*Haarwaschmittel*

Beispiel 6

Lösung, bestehend aus

50,0 g Dimethyl-carboxymethylen-propylenamidostearatbetain, 30%ig

2,0 g Chitosan (mit 90% freien Aminogruppen)

2,2 g Milchsäure, 90%ig

45,8 g Wasser, vollentsalzt

100,0 g

4

## Beispiel 7

Lösung, bestehend aus

40,0 g Dimethyl-carboxymethylen-propylenamidostearatbetain, 35%ig

2,0 g Chitosan (mit 90% freien Aminogruppen)

2,2 g Milchsäure, 90%ig

3,5 g Kokosfettsäurediäthanolamid

52,3 g Wasser, vollentsalzt
_____

100,0 g

## Beispiel 8

Lösung, bestehend aus

40,0 g Dimethyl-carboxymethylen-propylenamidostearatbetain, 35%ig

3,0 g Chitosan (mit 90% freien Aminogruppen)

7,6 g Ameisensäure, 10%ig

5,0 g Cetyltrimethylammoniumchlorid, 50%ig

44,4 g Wasser, vollentsalzt
_____

100,0 g

## Beispiel 9

Lösung (getrübt), bestehend aus

40,0 g Dimethyl-carboxymethylen-propylenamidostearatbetain, 35%ig

2,0 g Chitosan (mit 90% freien Aminogruppen)

2,2 g Milchsäure, 90%ig

5,0 g Cetyltrimethylammoniumchlorid, 50%ig

2,0 g Triäthylenglykoldistearat

48,8 g Wasser, vollentsalzt
_____

100,0 g

Mit jeweils etwa 15 bis 20 g der Haarwaschmittel gemäß den Beispielen 6 bis 9 wird das Haar in üblicher Weise gewaschen. Anschließend spült man mit Wasser aus. Das Haar ist hinsichtlich Griff, Glanz und Kämmbarkeit ausgezeichnet konditioniert.

## Beispiel 10

Lösung, bestehend aus

40,0 g Dimethyl-carboxymethylen-propylenamidostearatbetain, 35%ig

2,0 g Chitosan (mit 90% freien Aminogruppen)

5,06 g Ameisensäure, 10%ig

3,5 g Kokosfettsäurediäthanolamid

5

# 0 002 506

1,0  g  Haarorange II B (C.I. 76 540), 1%ige wäßrige Lösung

48,44 g  Wasser, vollentsalzt
_____

100,00 g

Mit etwa 15 bis 20 g wird das Haar einshampooniert. Nach einer Einwirkungszeit von 5 bis 10 Minuten spült man mit Wasser aus. Das Haar ist gelb-orange getönt und ausgezeichnet konditioniert.

Sämtliche in der Anmeldung angegebenen Prozentzahlen stellen Gewichtsprozente dar.

## Patentansprüche

1. Verwendung von Haarkur- und Haarwaschmitteln in Form einer wäßrigen Zubereitung auf üblicher kosmetischer Grundlage, gekennzeichnet durch einen zusätzlichen Gehalt an einem wasserlöslichen Salz von Chitosan.

2. Verwendung eines Haarkurmittels nach Anspruch 1 in Form einer wäßrigen Zubereitung auf der Grundlage von quaternären Ammoniumsalzen, Fettalkoholen, Fettsäureestern oder Paraffinkohlenwasserstoffverbindungen, gekennzeichnet durch einen zusätzlichen Gehalt an einem wasserlöslichen Salz von Chitosan.

3. Verwendung eines Haarwaschmittels nach Anspruch 1 in Form einer wäßrigen Zubereitung auf der Grundlage von nichtionischen, kationischen oder amphoteren Tensiden, gekennzeichnet durch einen zusätzlichen Gehalt an einem wasserlöslichen Salze von Chitosan.

4. Verwendung eines Mittels nach Anspruch 1 bis 3, dadurch gekennzeichnet, daß es das Chitosansalz der Ameisensäure, Essigsäure oder Milchsäure enthält.

5. Verwendung eines Mittels nach Anspruch 1 bis 4, dadurch gekennzeichnet, daß es gegebenenfalls zusätzlich direktziehende Farbstoffe enthält.

6. Verwendung nach Anspruch 1 bis 5, dadurch gekennzeichnet, daß man das Mittel auf das Haar aufträgt, eine ausreichende Zeit einwirken läßt und anschließend durch Ausspülen entfernt.

7. Verwendung nach Anspruch 1 bis 6, dadurch gekennzeichnet, daß man die wäßrige Zubereitung etwa 3 bis 10 Minuten lang einwirken läßt und anschließend mit Wasser ausspült.

## Revendications

1. Utilisation de compositions pour traiter et laver les cheveux sous forme d'une préparation aqueuse à base cosmétique usuelle, caractérisée par une teneur additionnelle en un sel soluble dans l'eau de chitosane.

2. Utilisation d'une composition pour le traitement des cheveux suivant la revendication 1 sous forme d'une préparation aqueuse à base de sels d'ammonium quaternaire, d'alcools gras, d'esters d'acides gras ou de composés d'hydrocarbures paraffiniques, caractérisée par une teneur additonnelle en un sel soluble dans l'eau de chitosane.

3. Utilisation d'une composition pour laver les cheveux suivant la revendication 1 sous forme d'une préparation aqueuse à base d'agents tensio-actifs non-ioniques, cationiques ou amphotères, caractérisée par une teneur additionnelle en un sel soluble dans l'eau de chitosane.

4. Utilisation d'une composition suivant l'une quelconque des revendications 1 à 3, caractérisée en ce qu'elle renferme le sel de chitosane de l'acide formique, de l'acide acétique ou de l'acide lactique.

5. Utilisation d'une composition suivant l'une quelconque des revendications 1 à 4, caractérisée en ce qu'elle renferme éventuellement en outre des colorants à effet direct.

6. Utilisation suivant l'une quelconque des revendications 1 à 5, caractérisée en ce qu'on applique la préparation sur les cheveux, la laisse agir pendant un laps de temps suffisant, puis l'enlève par rinçage.

7. Utilisation suivant l'une quelconque des revendications 1 à 6, caractérisée en ce qu'on laisse agir la préparation aqueuse pendant environ 3 à 10 minutes, puis on rince avec de l'eau.

## Claims

1. Use of hair treatment and hair-washing media in the form of an aqueous preparation on a conventional cosmetic basis, characterised by an additional content of a water-soluble chitosan salt.

2. Use of a hair treatment material according to claim 1 in the form of an aqueous preparation based on quarternary ammonium salts, fatty alcohols, fatty acid esters, or paraffin hydrocarbon compounds, characterised by an additional content of a water-soluble chitosan salt.

3. Use of a hair-washing medium according to claim 1 in the form of an aqueous preparation based on nonionic, cationic, or amphoteric surfactants, characterised by an additional content of a water-soluble chitosan salt.

4. Use of a medium according to claim 1 to 3, characterised in that it contains the chitosan salt of formic acid, acetic acid, or lactic acid.

5. Use of a medium according to claim 1 to 4, characterised in that if necessary it contains additional direct dyes.

6. Use according to claim 1 to 5, characterised in that the medium is applied to the hair, allowed to act for a sufficient time, and finally removed by rinsing.

7. Use according to claim 1 to 6, characterised in that the aqueous preparation is allowed to act for about 3 to 10 minutes and is then rinsed out with water.